Europäisches Patentamt

European Patent Office

Office européen des brevets

(11) Publication number: **0 347 057**
**A1**

## EUROPEAN PATENT APPLICATION

(21) Application number: **89305368.6**

(22) Date of filing: **26.05.89**

(51) Int. Cl.⁴: **A61K 39/395**

(30) Priority: **31.05.88 US 200755**

(43) Date of publication of application:
**20.12.89 Bulletin 89/51**

(84) Designated Contracting States:
**ES GR**

(71) Applicant: **Schering Biotech Corporation**
**901 California Avenue**
**Palo Alto California 94304-1104(US)**

(72) Inventor: **Abrams, John S.**
**1818 Oak Knoll Drive**
**Belmont California 94002(US)**

(74) Representative: **Ritter, Stephen David et al**
**Mathys & Squire 10 Fleet Street**
**London EC4Y 1AY(GB)**

(54) **Method of treating myeloid leukemias.**

(57) A method of treating myeloid leukemias is provided which comprises administering to a person an effective amount of an antagonist to GM-CSF. Preferably, the antagonist is a blocking monoclonal antibody specific for human GM-CSF, or a fragment or binding composition derived therefrom.

EP 0 347 057 A1

## METHOD OF TREATING MYELOID LEUKEMIAS

### Field of the Invention

The invention relates generally to a method for treating diseases caused or exacerbated by excessive production of granulocyte-macrophage colony stimulating factor (GM-CSF), and more particularly to a method of treating myeloid leukemias.

### BACKGROUND

Circulating blood cells are constantly replaced by newly developed cells. Replacement blood cells are formed in a process termed hematopoiesis in which at least eight mature blood cell types within two major lineages are produced: (1) myeloid lineages, which include red blood cells (erythrocytes), macrophages (monocytes), eosinophilic granulocytes, megakaryocytes (platelets), neutrophilic granulocytes, basophilic granulocytes (mast cells); and (2) lymphoid lineages, which include T lymphocytes, and B lymphocytes: Burgess and Nicola, Growth Factors and Stem Cells - (Academic Press, New York, 1983). Much of the control of blood cell formation is mediated by a group of interacting glycoproteins termed colony stimulating factors (CSFs). These glycoproteins are so named because of the in vivo and in vitro assays used to detect their presence. Techniques for the clonal culture of hematopoietic cells in semisolid culture medium have been especially important in the development of in vitro assays. In such cultures, individual progenitor cells (i.e., cells developmentally committed to a particular lineage, but still capable of proliferation) are able to proliferate to form a colony of maturing progeny in a manner which is believed to be essentially identical to the comparable process in vivo. The role of CSFs in hematopoiesis is the subject of many recent reviews, e.g. Metcalf, The Hemopoietic Colony Stimulating Factors (Elsevier, New York, 1984), Metcalf, Science, Vol. 1229, pgs. 16-22 1985), Nicola et al., Immunology Today, Vol. 5, pgs. 76-80 (1984), Whetton et al., TIBS, Vol. 11, pgs. 207-211 (1986), Clark and Kamen, Science, Vol. 236, pgs. 1229-1237 (1987), and Sachs, Science, Vol. 238, pgs. 1374-1379 (1987).

The detection, isolation and purification of these factors is extremely difficult, being frequently complicated by the nature of the biological fluids they are typically located in, and their very low concentrations. As more CSFs become available, primarily through molecular cloning, interest has heightened in finding clinical applications for them. Because of physiological similarities to hormones (e.g., soluble factors, growth mediators, action via cell receptors), potential uses of CSFs have been analogized to the current uses of hormones, e.g. Dexter, Nature, Vol. 321, pg. 198 (1986). Their use has been suggested for several clinical situations where the stimulation of blood cell generation would be desirable, such as rehabilitative therapy after chemotherapy or radiation therapy of tumors, treatment of myeloid hypoplasias, treatment of neutrophil deficiency, treatment to enhance hematopoietic regeneration following bone marrow transplantation, and treatment to increase host resistance to established infections, e.g. Dexter (cited above), Metcalf, Science (cited above), and Clark and Kamen (cited above). Recently, recombinant human GM-CSF has been shown to produce dose-dependent increases in circulating leukocyte count in severely leukopenic AIDS patients: Groopman, Cell, Vol. 50, pgs. 5-6 (1987).

CSFs are also believed to play a role in the development and progression of myeloid leukemias. Myeloid leukemias are clonal neoplasms of granulocyte-macrophage precursor cells, which fall into two major groups - chronic myeloid leukemia (CML) and acute myeloid leukemia (AML). CML is characterized by expansion in the marrow of the granulocyte-monocyte population at all stages of maturation with massive enlargement of hematopoietic populations in the spleen and blood. Whereas chemotherapy is successful in reducing the excessive size of the leukemic cell populations, conventional regimens have not succeeded in preventing terminal acute transformation (of progressively higher proportions of cells into immature or abnormal forms) or in extending the life spans of afflicted patients, Metcalf (cited above, 1984).

AML is characterized by an accumulation of immature granulocyte-monocyte blast cells with often little or no evidence of maturing granulocyte-monocyte cells. The disease primarily involves the bone marrow, and spleen enlargement usually is only moderate. Total blood nucleated cells may or may not be elevated but there is a high proportion of immature blast cells associated with relatively few mature cells. There is usually an associated anemia, thrombocytopenia and a relative absence in the marrow and peripheral blood of mature granulocytes and monocytes. Death usually results from uncontrollable hemorrhage or overwhelming infections; Metcalf (cited above, 1984).

It is believed that both forms of leukemia are driven by abnormal production of, or responsiveness to, colony stimulating factors, particularly GM-CSF. In particular, it has been shown that leukemic cells from some AML patients are capable of autonomous in vitro proliferation because they express GM-CSF constitutively, and that such autonomous proliferation can be inhibited by the addition of GM-CSF neutralizing antiserum; Young et al., Blood, Vol. 68, pgs. 1178-1181 (1986).

## SUMMARY OF THE INVENTION

The present invention relates to the treatment of myeloid leukemias, in particular AML, by blocking the ability of GM-CSF to stimulate cell growth. Such blocking could be carried out by the use of monoclonal antibodies specific for human GM-CSF. Accordingly, the availability of monoclonal antibodies capable of blocking GM-CSF activity could give rise to a new approach to treating myeloid leukemias.

The invention therefore provides a method of reducing effective GM-CSF concentrations by administering an effective amount of an antagonist to human GM-CSF. More particularly, the invention includes a method for treating myeloid leukemias by administering an effective amount of an antagonist to human GM-CSF. Preferably, the antagonists to GM-CSF are monoclonal antibodies capable of blocking the biological activity of GM-CSF, fragments thereof, or binding compositions derived therefrom by standard techniques. Most preferably, antagonists to GM-CSF are derived from the monoclonal antibodies, or the genes thereof, produced by hybridomas BVD2-5A2.4, BVD2-23B6.4, and BVD-21C11.3. These hybridomas are deposited at the American Type Culture Collection (ATCC) in Rockville, Maryland, under accession number HB9567, HB9568 and HB9569, respectively.

## DETAILED DESCRIPTION OF THE INVENTION

The invention is based on the discovery of monoclonal antibodies capable of blocking the biological activity of human GM-CSF, and the discovery that certain myeloid leukemias are caused by autocrine secretion of GM-CSF. The method of the invention comprises administering to a person an effective, or disease-ameliorating amount, of an antagonist to human GM-CSF. Preferably, the antagonists of the invention are derived from antibodies capable of blocking the biological activity of human GM-CSF.

Antibodies comprise an assembly of polypeptide chains linked together by disulfide bridges. Two major polypeptide chains, referred to as the light chain and the heavy chain, make up all major structural classes (isotypes) of antibody. Both heavy chains and light chains are further divided into subregions referred to as variable regions and constant regions. Heavy chains comprise a single variable region and three different constant regions, and light chains comprise a single variable region (different from that of the heavy chain) and a single constant region (different from those of the heavy chain). The variable regions of the heavy chain and light chain are responsible for the antibody's binding specificity.

As used herein, the term "heavy chain variable region" means a polypeptide (1) which is from 110 to 125 amino acids in length, and (2) whose amino acid sequence corresponds to that of a heavy chain of a monoclonal antibody of the invention, starting from the heavy chain's N-terminal amino acid. Likewise, the term "light chain variable region" means a polypeptide (1) which is from 95 to 115 amino acids in length, and (2) whose amino acid sequence corresponds to that of a light chain of a monoclonal antibody of the invention, starting from the light chain's N-terminal amino acid.

As used herein the term "monoclonal antibody" refers to homogenous populations of immunoglobulins which are capable of specifically binding to, and blocking the biological activity of, human GM-CSF.

As used herein the term "binding composition" means a composition comprising two polypeptide chains (1) which, when operationally associated, assume a conformation having high binding affinity for human GM-CSF, and (2) which are derived from a hybridoma producing monoclonal antibodies specific for, and capable of blocking the biological activity of, human GM-CSF. The term "operationally associated" is meant to indicate that the two polypeptide chains can be positioned relative to one another for binding by a variety of means, including association in a native antibody fragment, such as Fab or Fv, or by way of genetically engineered cysteine-containing peptide linkers at the carboxyl termini. Normally, the two polypeptide chains correspond to the light-chain variable region and heavy-chain variable region of a monoclonal antibody specific for human GM-CSF.

Preferably, antagonists of the invention are derived from monoclonal antibodies capable of blocking the biological activity of human GM-CSF. Such monoclonal antibodies are obtained by a double screening procedure. The first, by an indirect enzyme-linked immunoabsorbent assay (ELISA) or an immunometric ELISA screen, selects for antibodies specific for human GM-CSF. From this set, a second screen is used to assess the antibody's ability to block, or neutralize, the biological activity of human GM-CSF. Several biological assays are available for GM-CSF. In particular, bone marrow or umbilical cord blood assays in a semi-solid medium can be used, e.g. as disclosed by Metcalf, The Hemopoietic Colony Stimulating Factors - (Elsevier, Amsterdam, 1984), and the GM-CSF-dependent cell line KG-1 can be used, e.g. in such assays as are disclosed by Koeffler et al., Science, Vol. 200, pgs. 1153-1154 (1978), and by Lusis et al., Proc. Natl. Acad. Sci., Vol. 77, pgs. 5346-5350 (1980); the KG-1 cell line is available through the ATCC under accession number CCL 246.

Bone marrow and cord blood assays are carried out as follows. Bone marrow cells collected from patients with nonhematologic disease are layered over Ficoll (type 400, Sigma Chemical Co., St. Louis, MO) and centrifuged (600 x g, 20 min), and the cells at the interface removed. These cells are washed twice in Iscove's Modified Dulbecco's Medium containing 10% fetal calf serum (FCS) and resuspended in the same medium, and the adherent cells removed by adherence to plastic Petri dishes (adherent cells are frequently GM-CSF producing cells; see Metcalf (cited above)). The nonadherent cells are added at $10^5$ cells/ml to Iscove's Medium containing 20% FCS, 50 $\mu$M 2-mercaptoethanol, 0.9% methylcellulose and various concentrations of either supernatants known to contain colony stimulating activity or test supernatants. One ml aliquots are plated in 35 mm petri dishes and cultured at 37°C in a fully humidified atmosphere of 6% $CO_2$ in air. Three days after the initiation of the culture, 1 unit of erythropoietin is added to each plate. Granulocyte-macrophage colonies and erythroid bursts are scored at 10-14 days using an inverted microscope.

Cord blood cells collected in heparin are spun at 600 x g for 6 min. The white blood cells at the interface between the plasma and red blood cell peak are transferred to a tube containing 0.17 N ammonium chloride and 6% FCS. After 5 min on ice, the suspension is underlaid with 4 ml FCS and centrifuged for 6 minutes at 600 x g. The cell pellet is washed with Dulbecco's phosphate buffered saline and put through the Ficoll and plastic adherence steps as described above for bone marrow cells. The low density nonadherent cells are collected and placed at $10^5$ cells/culture in the semi-solid culture medium as described above.

At the end of the assays, the cellular composition is determined after applying the individual colonies to glass slides and staining with Wright-Geimsa. Eosinophils are determined by staining with Luxol Fast Blue, e.g. Johnson, G. and Metcalf, D., Exp. Hematol., Vol. 8, pgs. 549-561 (1980).

Hybridomas of the invention are produced by well-known techniques. Usually, an immortalizing cell line is fused with a B-lymphocyte that produces the desired antibody. Alternatively, non-fusion techniques for generating immortal antibody-producing cell lines are possible, and come within the purview of the present invention; e.g. virally induced transformation: Casali et al., "Human Monoclonals from Antigen-Specific Selection of B Lymphocytes and Transformation by EBV," Science, Vol. 234, pgs. 476-479 (1986). Immortalizing cell lines are usually transformed mammalian cells, particularly myeloma cells of rodent, bovine, or human origin. Most frequently, rat or mouse myeloma cell lines are employed as a matter of convenience and availability.

Techniques for obtaining the appropriate lymphocytes from mammals injected with the target antigen are well known. Generally, peripheral blood lymphocytes (PBLs) are used if cells of human origin are desired, whereas spleen cells or lymph node cells are used if non-human mammalian sources are desired. A host mammal is injected with repeated dosages of the purified antigen, and the mammal is permitted to generate the desired antibody-producing cells before these are harvested for fusion with the immortalizing cell line. Techniques for fusion are also well known in the art, and in general involve mixing the cells with a fusing agent, such as polyethylene glycol. Hybridomas are selected by standard procedures, such as HAT selection. From among these hybridomas, those secreting the desired antibody are selected by assaying their culture medium by standard immunoassays, such as Western blotting, ELISA, RIA, or the like. Antibodies are recovered from the medium using standard protein purification techniques, e.g. Tijssen, Practice and Theory of Enzyme Immunoassays (Elsevier, Amsterdam, 1985). Many references are available for guidance in applying any of the above techniques: e.g. Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrel, Monoclonal Hybridoma Antibodies: Techniques and Applications (CRC Press, Boca Raton, FL, 1982); and the like.

The use and generation of fragments of antibodies is also well known; e.g. Fab fragments: Tijssen, Practice and Theory of Enzyme Immunoassays (Elsevier, Amsterdam, 1985); and Fv fragments: Hochman et al. Biochemistry, Vol. 12, pgs. 1130-1135 (1973), Sharon et al., Biochemistry, Vol. 15, pgs. 1591-1594 (1976) and Ehrlich et al., U.S. Patent 4,355,023; and antibody half molecules: Auditore-Hargreaves, U.S. Patent 4,470,925. Moreover, such compounds and compositions of the invention can be used to construct bi-specific antibodies by known techniques, e.g., by further fusions of hybridomas (i.e. to form so-called quadromas), Reading, U.S. Patent 4,474,493, or by chemical reassociation of half molecules, Brennan et al., Science, Vol. 229, pgs. 81-83 (1985).

Hybridomas and monoclonal antibodies of the invention are produced against either glycosylated or unglycosylated versions of recombinantly produced mature human GM-CSF. Generally, unglycosylated versions of human GM-CSF are produced in E. coli, and glycosylated versions are produced in mammalian cell hosts, e.g. CV1 or COS monkey cells, mouse L cells, or the like. Recombinantly produced mature human GM-CSF is produced by introducing an expression vector into a host cell using standard protocols: e.g. Maniatis et al., Molecular Cloning: A Laboratory Manual (Cold Spring Harbor Laboratory, New York, 1982); Okayama and Berg, Mol. Cell. Biol., Vol. 2, pgs. 161-170 (1982) and Vol. 3, pgs. 280-289 (1983); Hamer, Genetic Engineering, Vol. 2, pgs. 83-100 (1980) and U.S. Patent 4,599,308; Kaufman et al., Mol. Cell. Biol., Vol. 2, pgs. 1304-1319 (1982); or the like.

Construction of bacterial or mammalian expression vectors is well known in the art, once the nucleotide sequence encoding a desired protein is known or otherwise available; e.g. DeBoer in U.S. Patent 4,551,433 discloses promoters for use in bacterial expression vectors; Goeddel et al., in U.S. Patent 4,601,980, and Riggs, in U.S. Patent 4,431,739 disclose the production of mammalian proteins by E. coli expression systems; and Riggs (cited above), Ferretti et al., Proc. Natl. Acad. Sci., Vol. 83, pgs. 599-603 (1986), Sproat et al., Nucleic Acids Research, Vol. 13, pgs. 2959-2977 (1985), and Mullenbach et al., J. Biol. Chem., Vol. 261, pgs. 719-722 (1986) disclose how to construct synthetic genes for expression in bacteria. Accordingly, these references are incorporated by reference. The amino acid sequence of human GM-CSF is disclosed by Lee et al., Proc. Natl. Acad. Sci., Vol. 82, pgs. 4360-4364 (1985), and the pcD vector carrying the cDNA encoding human GM-CSF disclosed therein is deposited in the NIH Repository of the ATCC under accession numbers 57594 and 57595. Production of human GM-CSF in yeast is disclosed by Miyajima et al., EMBO J., Vol. 5, pgs. 1193-1197 (1986), and by Cantrell et al., Proc. Natl. Acad. Sci., Vol. 82, pgs. 6250-6254 (1985). Production of human GM-CSF in E. coli is disclosed by Libby et al., DNA, Vol. 6, pgs. 221-229 (1987).

Antibodies and antibody fragments characteristic of hybridomas of the invention can also be produced by recombinant means by extracting messenger RNA, constructing a cDNA library, and selecting clones which encode segments of the antibody molecule: e.g. Wall et al., Nucleic Acids Research, Vol. 5, pgs. 3113-3128 (1978); Zakut et al., Nucleic Acids Research, Vol. 8, pgs. 3591-3601 (1980); Cabilly et al., Proc. Natl. Acad. Sci., Vol. 81, pgs. 3273-3277 (1984); Boss et al., Nucleic Acids Research, Vol. 12, pgs. 3791-3806 (1984); Amster et al., Nucleic Acids Research, Vol. 8, pgs. 2055-2065 (1980); and Moore et al., U.S. Patent 4,642,334. In particular, such techniques can be used to produce interspecific monoclonal antibodies, wherein the binding region of one species is combined with a non-binding region of the antibody of another species to reduce immunogenicity, e.g. Liu et al., Proc. Natl. Acad. Sci., Vol. 84, pgs. 3439-3443 (1987).

Antagonists of the invention are administered as a pharmaceutical composition for treating myeloid leukemias. Such compositions contain an effective amount of at least one of the monoclonal antibodies of the invention, or fragments thereof, in a pharmaceutical carrier. A pharmaceutical carrier can be any compatible, non-toxic substance suitable for delivering the compositions of the invention to a patient. Generally, compositions useful for parenteral administration of such drugs are well known, e.g. Remington's Pharmaceutical Science, 15th Ed. (Mack Publishing Company, Easton, PA 1980). Alternatively, compositions of the invention may be introduced into a patient's body by an implantable drug delivery system, e.g. Urquhart et al., Ann. Rev. Pharmacol. Toxicol., Vol. 24, pgs. 199-236 (1984).

When administered parenterally the antibodies or fragments will be formulated in a unit dosage injectable form (solution, suspension, emulsion) in association with a pharmaceutically acceptable pharmaceutical carrier. Such carriers are inherently nontoxic and nontherapeutic. Examples of such carriers are normal saline, Ringer's solution, dextrose solution, and Hank's solution. Nonaqueous carriers such as fixed oils and ethyl oleate may also be used. A preferred carrier is 5% dextrose/saline. The carrier may contain minor amounts of additives such as substances that enhance isotonicity and chemical stability, e.g., buffers and preservatives. The antibody is preferably formulated in purified form substantially free of aggregates and other proteins at concentrations of about 5 to 30 mg/ml, preferably 10 to 20 mg/ml.

Selecting an administration regimen for an antagonist depends on several factors, including the serum turnover rate of the antagonist, the circulating, extracellular level of GM-CSF associated with the leukemia, the immunogenicity of the antagonist, the accessibility of the target GM-CSF (e.g. if non-serum GM-CSF is to be blocked), the relative affinity of GM-CSF to its receptor(s) versus GM-CSF to the antagonist, and the like. Preferably, an administration regimen maximizes the amount of antagonist delivered to the patient consistent with an acceptable level of side effects. Accordingly, the amount of antagonist delivered depends in part on the particular antagonist and the severity of the disease being treated. Guidance in selecting appropriate doses is found in the literature on therapeutic uses of antibodies; e.g. Bach et al., chapter 22, in Ferrone et al., eds., Handbook of Monoclonal Antibodies (Noges Publications, Park Ridge, NJ, 1985); and Russell, pgs. 303-357, and Smith et al., pgs. 365-389, in Haber et al., eds. Antibodies in Human Diagnosis and Therapy (Raven Press, New York, 1977). Preferably, whenever the antagonist comprises monoclonal antibodies or Fab-sized fragments thereof (including binding compositions), the dose is in the range of about 1-20 mg/kg per day. More preferably the dose is in the range of abut 1-10 mg/kg per day.

In some cases the immunogenicity of the antibodies or fragments can be reduced by appropriate derivatization, e.g. with polyethylene glycol, or the like; Beauchamp et al., Anal. Biochem., Vol. 131, pgs. 25-33 (1983); and Abuchowski et al., J. Biol. Chem., Vol. 252, pgs. 3578-3581 and 3582-3586 (1977).

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments were chosen and described in order to best explain the principles of the invention and its practical application to thereby enable others skilled in the art to best utilize the invention in various embodiments and with various modifications as are suited to the particular use contemplated. It is intended that the scope of the invention be defined by the claims appended hereto.

Applicants have deposited hybridomas BVD-5A2.4, BVD2-23B6.4, and BVD2-21C11.3 with the American Type Culture Collection, Rockville, Maryland, USA (ATCC), under accession numbers HB9567, HB9568, and HB9569, respectively. These deposits were made under conditions as provided under ATCC's Agreement for Culture Deposit for Patent Purposes, which assures that the deposits will be made available to the US Commissioner of Patents and Trademarks pursuant to 35 U.S.C. 122 and 37 C.F R. 1.14, and will be made available to the public upon issue of a U.S. patent, which requires that the deposits be maintained. Availability of the deposited strains is not to be construed as a license to practise the invention in contravention of the rights granted under the authority of any government in accordance with its patent laws.

The deposit has been modified to conform to the requirements of the Budapest Treaty on the Deposit of Microorganisms.

## Claims

1. A method of treating myeloid leukemia in a person comprising administering to said person an effective amount of an antagonist to human GM-CSF.

2. The method of claim 1 wherein said antagonist to human GM-CSF is selected from a monoclonal antibody capable of blocking the biological activity of human GM-CSF, a fragment of the monoclonal antibody capable of blocking the biological activity of human GM-CSF, and a binding composition comprising the heavy-chain variable region and light-chain variable region of the monoclonal antibody capable of blocking the biological activity of human GM-CSF.

3. The method of claim 2 wherein said monoclonal antibody is produced by a hybridoma selected from BVD2-5A2.4, BVD2-23B6.4, and BVD2-21C11.3.

4. The method of claim 2 wherein said step of administering includes intravenous delivery of said antagonist at a concentration in the range of about 1-20 mg/ml.

5. The method of claim 4 wherein said step of administering further includes intravenous delivery of an amount of said antagonist in the range of about 1-20 mg/kg body weight of said person per day.

6. The method of claim 5 wherein said myeloid leukemia is acute myeloid leukemia.

7. The use of an antagonist to human GM-CSF in the manufacture of a pharmaceutical composition for use in carrying out the method of any one of Claims 1 to 6.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 265 384 (SANDOZ-PATENT-GMBH) * pages 7,8 * --- | 1-7 | A 61 K 39/395 |
| X,D | BLOOD vol. 68, no. 5, November 1986, pages 1178-1181; D. C. YOUNG et al.: "Autocrine Secretion of GM-SCF in Acute Myeloblastic Leukemia" * the whole document * --- | 1-7 | |
| X,P | CANCERLIT abstract ICDB 89050187, Exp. Hematol. vol. 16, no. 6, 1988; P. MANNONI et al.: "Role of hemopoietic growth facotrs in the proliferation of human acute myeloid leukemias (Meeting abstract)" --- | 1-7 | |
| X,P | MEDLINE abstract MED/88231767, Leukemia vol. 2, no. 6, pages 334-342, 1988; U. DUHRSEN: "In vitro growth patterns and autocrine production of hemopoietic colony stimulating factors: Analysis of leukemic populations arising in irradiated mice from cells of an injected factor-dependent continuous cell line" --- | 1-7 | TECHNICAL FIELDS SEARCHED (Int. Cl.4) A 61 K C 12 P |
| A,P | CANCERLIT abstracts ICDB/89050230, Exp. Hematol, vol. 16, no. 6, page 476, 1988; M. KOHASHI et al: "Concentration of GM-CSF in sera from patients with hematological disorders (Meeting abstract)" --- -/- | 1-7 | |

The present search report has been drawn up for all claims

| Place of search | Date of completion of the search | Examiner |
|---|---|---|
| BERLIN | 07-09-1989 | AVEDIKIAN P.F. |

**European Patent Office**

Application Number

EP 89 30 5368

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | WO-A-8 600 639 (SANDOZ AG) <br> * page 5 * <br> --- | 1-7 | |
| A | EP-A-0 227 367 (KIRI-AMGEN) <br> * the whole document * <br> ----- | 1-7 | |
| | | | **TECHNICAL FIELDS SEARCHED (Int. Cl.4)** |

The present search report has been drawn up for all claims

| Place of search | Date of completion of the search | Examiner |
|---|---|---|
| BERLIN | 07-09-1989 | AVEDIKIAN P.F. |

EPO FORM 1503 03.82 (P0401)